# EUROPÄISCHE PATENTSCHRIFT

(11) **EP 0 966 449 B1**
(45) Veröffentlichungstag und Bekanntmachung des Hinweises auf die Patenterteilung: **16.04.2003**
(21) Anmeldenummer: 98912353.4
(22) Anmeldetag: 20.02.1998
(51) Int. Cl.: C07D 243/08, A61K 7/13

(54) **1,4-DIAZACYCLOHEPTAN-DERIVATE UND DEREN VERWENDUNG IN OXIDATIONSHAARFÄRBEMITTELN**
1,4-DIAZACYCLOHEPTANE DERIVATIVES AND THEIR USE IN HAIR OXIDATION DYES
DERIVES DE 1,4-DIAZACYCLOHEPTANE ET LEUR UTILISATION DANS DES COLORANTS CAPILLAIRES D'OXYDATION

(30) Priorität: 26.02.1997 DE 19707545
(43) Veröffentlichungstag der Anmeldung: 29.12.1999
(73) Patentinhaber: Henkel Kommanditgesellschaft auf Aktien, 40589 Düsseldorf-Holthausen (DE)
(72) Erfinder: ROSE, David, D-40723 Hilden (DE); HÖFFKES, Horst, D-40595 Düsseldorf (DE); MEINIGKE, Bernd, D-51381 Leverkusen (DE)
(86) Internationale Anmeldenummer: EP9800986
(87) Internationale Veröffentlichungsnummer: WO98038175

(56) Entgegenhaltungen:
- EP-A- 0 008 079
- WO-A-98/01434

## Beschreibung

Die Erfindung betrifft neue Diazacycloheptan-Derivate, deren Verwendung zum Färben von Keratinfasern sowie diese Verbindungen enthaltende Färbemittel.

Für das Färben von Keratinfasern, insbesondere menschlichen Haaren, spielen die sogenannten Oxidationsfärbemittel wegen ihrer intensiven Farben und guten Echtheitseigenschaften eine bevorzugte Rolle. Solche Färbemittel enthalten Oxidationsfarbstoffvorprodukte, sogenannte Entwicklerkomponenten und Kupplerkomponenten. Die Entwicklerkomponenten bilden unter dem Einfluß von Oxidationsmitteln oder von Luftsauerstoff untereinander oder unter Kupplung mit einer oder mehreren Kupplerkomponenten die eigentlichen Farbstoffe aus.

Gute Oxidationsfarbstoffvorprodukte müssen in erster Linie folgende Voraussetzungen erfüllen: Sie müssen bei der oxidativen Kupplung die gewünschten Farbnuancen in ausreichender Intensität und Echtheit ausbilden. Sie müssen ferner ein gutes Aufziehvermögen auf die Faser besitzen, wobei insbesondere bei menschlichen Haaren keine merklichen Unterschiede zwischen strapaziertem und frisch nachgewachsenem Haar bestehen dürfen (Egalisiervermögen). Sie sollen beständig sein gegen Licht, Wärme und den Einfluß chemischer Reduktionsmittel, z. B. gegen Dauerwellflüssigkeiten. Schließlich sollen sie - falls als Haarfärbemittel zur Anwendung kommend - die Kopfhaut nicht zu sehr anfärben, und vor allem sollen sie in toxikologischer und dermatologischer Hinsicht unbedenklich sein.

Als Entwicklerkomponenten werden üblicherweise primäre aromatische Amine mit einer weiteren, in para- oder ortho-Position befindlichen, freien oder substituierten Hydroxy- oder Aminogruppe, Diaminopyridinderivate, heterocyclische Hydrazone, 4-Aminopyrazolonderivate sowie 2,4,5,6-Tetraaminopyrimidin und dessen Derivate eingesetzt.

Spezielle Vertreter sind beispielsweise p-Phenylendiamin, p-Toluylendiamin, 2,4,5,6-Tetraaminopyrimidin, p-Aminophenol, N,N-Bis(2-hydroxyethyl)-p-phenylendiamin, 2-(2,5-Diaminophenyl)-ethanol, 2-(2,5-Diaminophenoxy)-ethanol, 1-Phenyl-3-carboxyamido-4-amino-pyrazolon-5, 4-Amino-3-methylphenol, 2-Aminomethyl-4-aminophenol, 2-Hydroxy-4,5,6-triaminopyrimidin, 2,4-Dihydroxy-5,6-diaminopyrimidin. 2,5,6-Triaminohydroxypyrimidin und 1,3-N,N'-Bis(2'-hydroxyethyl)-N,N'-bis(4-aminophenyl)-diamino-propan-2-ol.

Als Kupplerkomponenten werden in der Regel m-Phenylendiaminderivate, Naphthole, Resorcin und Resorcinderivate, Pyrazolone und m-Aminophenole verwendet. Als Kupplersubstanzen eignen sich insbesondere 1-Naphthol, 1,5-, 2,7- und 1,7-Dihydroxynaphthalin, 5-Amino-2-methylphenol, m-Aminophenol, Resorcin, Resorcinmonomethylether, m-Phenylendiamin, 1-Phenyl-3-methyl-pyrazolon-5, 2,4-Dichlor-3-aminophenol. 1,3-Bis-(2,4-diaminophenoxy)-propan, 2-Chlor-resorcin, 4-Chlorresorcin, 2-Chlor-6-methyl-3-aminophenol, 2-Amino-4-hydroxypyridin, 2-Methylresorcin und 5-Methylresorcin.

Allein mit einer Entwicklerkomponente oder einer speziellen Kuppler/Entwickler-Kombination gelingt es in der Regel nicht, eine auf dem Haar natürlich wirkende Farbnuance zu erhalten. In der Praxis werden daher üblicherweise Kombinationen verschiedener Entwicklerkomponenten und Kupplerkomponenten eingesetzt. Es besteht daher ständig Bedarf an neuen, verbesserten Farbstoff-Komponenten.

Es war daher die Aufgabe der vorliegenden Erfindung, neue Entwickler-Komponenten zu finden, die die an Oxidationsfarbstoffvorprodukte zu stellenden Anforderungen in besonderem Maße erfüllen.

Es wurde nun gefunden, daß bestimmte, bisher nicht bekannte 1,4-Diazacycloheptan-Derivate die an Entwicklerkomponenten gestellten Anforderungen in besonders hohem Maße erfüllen. So werden unter Verwendung dieser Entwicklerkomponenten mit den meisten bekannten Kupplerkomponenten brillante Farbnuancen, insbesondere im Braun- und Blau-Bereich, erhalten, die außerordentlich licht- und waschecht sind. Weiterhin zeichnen sich die erzielten Färbungen durch außerordentliche Kaltwellechtheit und Wärmestabilität, sowie durch eine hervorragende Egalisierung aus.

Ein erster Gegenstand der vorliegenden Erfindung sind daher
1,4-Diazacycloheptan-Derivate der allgemeinen Formel (I), 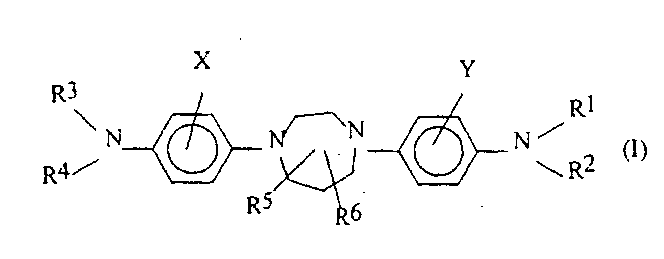 in der
- R¹, R², R³ und R⁴ unabhängig voneinander stehen für Wasserstoff, eine C₁₋₄-Alkyl- oder Hydroxyalkylgruppe oder eine C₂₋₄-Dihydroxyalkylgruppe,
- X und Y unabhängig voneinander stehen für Wasserstoff, Chlor, Fluor, eine C₁₋₄-Alkyl-. -Hydroxyalkyl-, -Aminoalkyl- oder -Alkoxygruppe, eine C₂₋₄-Dihydroxyalkylgruppe oder eine Allylgruppe und
- R⁵ und R⁶ unabhängig voneinander stehen für Wasserstoff oder eine C₁₋₄- Alkylgruppe.

Diese Verbindungen lassen sich mit bekannten organischen Synthesemethoden herstellen. Bezüglich der Einzelheiten wird ausdrücklich auf die im Beispielteil ausführlich dargestellten Synthesebeispiele verwiesen.

Besonders überraschend ist die hervorragende Eignung dieser Verbindungen als Entwickler-Komponente für Oxidationsfärbemittel auch deshalb, weil die analogen Piperidino-Verbindungen keine nennenswerten Entwickler-Eigenschaften aufweisen.

Da es sich bei allen erfindungsgemäßen Substanzen um Amino-Verbindungen handelt, lassen sich aus diesen in üblicher Weise die bekannten Säureadditionssalze herstellen. Alle Aussagen dieser Schrift und demgemäß der beanspruchte Schutzbereich beziehen sich daher sowohl auf die in freier Form vorliegenden 1,4-Diazacycloheptan-Derivate gemäß Formel (I) als auch auf deren wasserlösliche, physiologisch verträgliche Salze. Beispiele für solche Salze sind die Hydrochloride, die Hydrobromide, die Sulfate, die Phosphate, die Acetate, die Propionate, die Citrate und die Lactate.

Als erfindungsgemäß besonders geeignet haben sich die 1,4-Diazacycloheptan-Derivate gemäß Formel (I) erwiesen, bei denen beide Reste R⁵ und R⁶ am 1,4-Diazacycloheptan-Ring Wasserstoff sind.

Ebenfalls erfindungsgemäß bevorzugt sind solche Verbindungen gemäß Formel (I), bei denen mindestens drei, insbesondere alle vier, Gruppen R¹, R², R³ und R⁴ für Wasserstoff stehen.

Schließlich haben sich auch diejenigen 1,4-Diazacycloheptan-Derivate gemäß Formel (1) als erfindungsgemäß besonders geeignet erwiesen, bei denen die beiden Substituenten X und Y an den beiden aromatischen Ringen unabhängig voneinander stehen für Wasserstoff, Fluor, Chlor oder eine C₁₋₄-Alkylgruppe. Wasserstoff sowie Methylgruppen haben sich als ganz besonders vorteilhafte Gruppen X und Y erwiesen.

Besonders hervorragend im Sinne der Erfindung geeignete Substanzen sind
- N,N'-Bis(4-aminophenyl)-1,4-diazacycloheptan,
- N,N'-Bis(4-amino-2-methyl-phenyl)-1,4-diazacycloheptan und
- N,N'-Bis(4-arnino-3-methyl-phenyl)-1,4-diazacycloheptan.

Unter diesen Verbindungen sind wiederum N,N'-Bis(4-aminophenyl)-1,4-diazacycloheptan und N,N'-Bis(4-amino-3-methyl-phenyl)-1,4-diazacycloheptan bevorzugt.

Ein zweiter Gegenstand der vorliegenden Erfindung ist die Verwendung der vorgenannten 1,4-Diazacycloheptan-Derivate als Entwickler-Komponente in Oxidationshaarfärbemitteln.

Ein dritter Gegenstand der vorliegenden Erfindung sind schließlich Oxidationsfärbemittel zum Färben von Keratinfasern enthaltend Kupplerkomponenten und Entwicklerkomponenten in einem wasserhaltigen Träger, die als Entwickler-Komponente eines der vorgenannten 1,4-Diazacycloheptan-Derivate enthalten.

Unter Keratinfasern sind dabei Pelze, Wolle, Federn und insbesondere menschliche Haare zu verstehen. Obwohl die erfindungsgemäßen Oxidationsfärbemittel in erster Linie zum Färben von Keratinfasern geeignet sind, steht prinzipiell einer Verwendung auch auf anderen Gebieten, insbesondere in der Farbphotographie, nichts entgegen.

Die erfindungsgemäßen Oxidationsfärbemittel enthalten die erfindungsgemäßen Entwickler-Komponenten und können gewünschtenfalls noch weitere Entwickler-Komponenten sowie Kuppler-Komponenten enthalten. Bezüglich der weiteren Entwickler- und Kupplerkomponenten wird auf die zu Beginn der Beschreibung aufgeführten Substanzen verwiesen, die bevorzugte weitere Farbstoffkomponenten darstellen.

Erfindungsgemäß bevorzugte Entwicklerkomponenten sind p-Phenylendiamin, p-Toluylendiamin, p-Aminophenol, o-Aminophenol, 1-(2'-Hydroxyethyl)-2,5-diaminobenzol, N,N-Bis-(2-hydroxy-ethyl)-p-phenylendiamin, 2-(2,5-Diaminophenöxy)-ethanol, 1-Phenyl-3-carboxyamido-4-amino-pyrazolon-5, 4-Amino-3-methylphenol, 2,4,5,6-Tetraaminopyrimidin, 2-Hydroxy-4,5,6-triaminopyrimidin, 4-Hydroxy-2,5,6-triaminopyrimidin, 2,4-Dihydroxy-5,6-diaminopyrimidin, 2-Dimethylamino-4,5,6-triaminopyrimidin, 2-Hydroxyethylaminomethyl-4-amino-phenol, 4,4'-Diaminodiphenylamin, 4-Amino-3-fluorphenol, 2-Aminomethyl-4-amino-phenol, 2-Hydroxymethyl-4-aminophenol, Bis-(2-hydroxy-5-aminophenyl)-methan, 1,4-Bis-(4-aminophenyl)-diazacycloheptan, 1,3-Bis(N(2-hydroxyethyl)-N-(4-aminophenylamino))-2-propanol, 4-Amino-2-(2-hydroxyethoxy)-phenol sowie 4,5-Diaminopyrazol-Derivate nach EP 0 740 741 bzw. WO 94/08970 wie z.B. 4,5-Diamino-1-(2'-hydroxyethyl)-pyrazol.

Ganz besonders bevorzugte weitere Entwicklerkomponenten sind 2,4,5,6-Tetraminopyrimidin, 1-(2'-Hydroxyethyl)-2,5-diaminobenzol, 3-Methyl-4-aminophenol, o-Aminophenol, 2-Aminomethyl- und 2-Hydroxymethyl-4-aminophenol.

Erfindungsgemäß bevorzugte Kupplerkomponenten sind 1-Naphthol, Pyrogallol, 1,5-, 2,7- und 1,7-Dihydroxynaphthalin, o-Aminophenol, 5-Amino-2-methylphenol, m-Aminophenol, Resorcin, Resorcinmonomethylether, m-Phenylendiamin, 1-Phenyl-3-methyl-pyrazolon-5, 2,4-Dichlor-3-aminophenol, 1,3-Bis-(2,4-diaminophenoxy)-propan, 4-Chlorresorcin, 2-Chlor-6-methyl-3-aminophenol, 2-Methylresorcin, 5-Methylresorcin, 2,5-Dimethylresorcin, 2,6-Dihydroxypyridin, 2,6-Diaminopyridin, 2-Amino-3-hydroxypyridin, 2,6-Dihydroxy-3,4-diaminopyridin, 3-Amino-2-methylamino-6-methoxypyridin, 4-Amino-2-hydroxytoluol, 2,6-Bis-(2-hydroxyethylamino)-toluol, 2,4-Diaminophenoxyethanol, 1-Methoxy-2-amino-4-(2-hydroxyethylamino)-benzol, 2-Methyl-4-chlor-5-amino-phenol, 6-Methyl-1,2,3,4-tetrahydro-chinoxalin, 3,4-Methylendioxyphenol, 3,4-Methylendioxyanilin, 2.6-Dimethyl-3-amino-phenol, 3-Amino-6-methoxy-2-methylaminophenol, 2-Hydroxy-4-aminophenoxyethanol, 2-Methyl-5-(2-hydroxyethylamino)-phenol und 2,6-Dihydroxy-3,4-dimethylpyridin.

Besonders bevorzugte Kupplerkomponenten sind 1-Naphthol, Resorcin, 1,3-Bis(2,4-diaminophenoxy)propan, 2-Hydroxy-4-aminophenoxyethanol, 4-Chlorresorcin, 2,4-Diaminophenoxyethanol, 2-Methyl-resorcin, 2-Methyl-5(2-hydroxyethylamino)-phenol, 3-Amino-2-methylamino-6-methoxy-pyridin.

Diese weiteren Entwickler- und Kupplerkomponenten werden üblicherweise in freier Form eingesetzt. Bei Substanzen mit Aminogruppen kann es aber bevorzugt sein, sie in Salzform, insbesondere in Form der Hydrochloride und Sulfate, einzusetzen.

Die erfindungsgemäßen Haarfärbemittel enthalten sowohl die Entwicklerkomponenten als auch die Kupplerkomponenten bevorzugt in einer Menge von 0,005 bis 20 Gew.-%, vorzugsweise 0,1 bis 5 Gew.-%, jeweils bezogen auf das gesamte Oxidationsfärbemittel. Dabei werden Entwicklerkomponenten und Kupplerkomponenten im allgemeinen in etwa molaren Mengen zueinander eingesetzt. Wenn sich auch der molare Einsatz als zweckmäßig erwiesen hat, so ist ein gewisser Überschuß einzelner Oxidationsfarbstoffvorprodukte nicht nachteilig, so daß Entwicklerkomponenten und Kupplerkomponenten in einem Mol-Verhältnis von 1 : 0.5 bis 1 : 3, insbesondere 1:1 bis 1:2, enthalten sein können.

Direktziehende Farbstoffe sind üblicherweise Nitrophenylendiamine, Nitroaminophenole, Azofarbstoffe, Anthrachinone oder Indophenole. Bevorzugte direktziehende Farbstoffe sind die unter den internationalen Bezeichnungen bzw. Handelsnamen HC Yellow 2, HC Yellow 4, HC Yellow 5, HC Yellow 6, Basic Yellow 57, Disperse Orange 3, HC Red 3, HC Red BN, Basic Red 76, HC Blue 2, HC Blue 12, Disperse Blue 3, Basic Blue 99, HC Violet 1, Disperse Violet 1, Disperse Violet 4, Disperse Black 9, Basic Brown 16 und Basic Brown 17 bekannten Verbindungen sowie 4-Amino-2-nitrodiphenylamin-2'-carbonsäure, 6-Nitro-1,2,3,4-tetrahydrochinoxalin, Hydroxyethyl-2-nitrotoluidin, Pikraminsäure, 2-Amino-6-chloro-4-nitrophenol 4-Ethylamino-3-nitrobenzoesäure und 2-Chloro-6-ethylamino-1-hydroxy-4-nitrobenzol. Die erfindungsgemäßen Mittel gemäß dieser Ausführungsform enthalten die direktziehenden Farbstoffe bevorzugt in einer Menge von 0,01 bis 20 Gew.-%, bezogen auf das gesamte Färbemittel.

Weiterhin können die erfindungsgemäßen Zubereitungen auch in der Natur vorkommende Farbstoffe wie beispielsweise Henna rot, Henna neutral, Henna schwarz, Kamillenblüte, Sandelholz, schwarzen Tee, Faulbaumrinde, Salbei, Blauholz, Krappwurzel, Catechu, Sedre und Alkannawurzel enthalten.

Weitere in den erfindungsgemäßen Färbemitteln enthaltene Farbstoffkomponenten können auch Indole und Indoline, sowie deren physiologisch verträgliche Salze, sein. Bevorzugte Beispiele sind 5,6-Dihydroxyindol, N-Methyl-5,6-dihydroxyindol, N-Ethyl-5,6-dihydroxyindol, N-Propyl-5,6-dihydroxyindol, N-Butyl-5,6-dihydroxyindol, 6-Hydroxyindol, 6-Aminoindol und 4-Aminoindol. Weiterhin bevorzugt sind 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin, 6-Hydroxyindolin, 6-Aminoindolin und 4-Aminoindolin.

Es ist nicht erforderlich, daß die Oxidationsfarbstoffvorprodukte oder die fakultativ enthaltenen direktziehenden Farbstoffe jeweils einheitliche Verbindungen darstellen. Vielmehr können in den erfindungsgemäßen Haarfärbemitteln, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z.B. toxikologischen, ausgeschlossen werden müssen.

Bezüglich der in den erfindungsgemäßen Haarfarbe- und -tönungsmitteln einsetzbaren Farbstoffe wird weiterhin ausdrücklich auf die Monographie Ch, Zviak, The Science of Hair Care, Kapitel 7 (Seiten 248-250; direktziehende Farbstoffe) sowie Kapitel 8, Seiten 264-267; Oxidationsfarbstoffvorprodukte), erschienen als Band 7 der Reihe "Dermatology" (Hrg.: Ch., Culnan und H. Maibach), Verlag Marcel Dekker Inc., New York, Basel, 1986, sowie das "Europäische Inventar der Kosmetik-Rohstoffe", herausgegeben von der Europäischen Gemeinschaft, erhältlich in Diskettenform vom Bundesverband Deutscher Industrie- und Handelsunternehmen für Arzneimittel, Reformwaren und Körperpflegemittel e.V., Mannheim, Bezug genommen.

Zur Herstellung der erfindungsgemäßen Färbemittel werden die Oxidationsfarbstoffvorprodukte in einen geeigneten wasserhaltigen Träger eingearbeitet. Zum Zwecke der Haarfärbung sind solche Träger z.B. Cremes, Emulsionen, Gele oder auch tensidhaltige schäumende Lösungen, z.B. Shampoos, Schaumaerosole oder andere Zubereitungen, die für die Anwendung auf dem Haar geeignet sind.

Die erfindungsgemäßen Färbemittel können weiterhin alle für solche Zubereitungen bekannten Wirk-, Zusatz- und Hilfsstoffe enthalten. In vielen Fällen enthalten die Färbemittel mindestens ein Tensid, wobei prinzipiell sowohl anionische als auch zwitterionische, ampholytische, nichtionische und kationische Tenside geeignet sind. In vielen Fällen hat es sich aber als vorteilhaft erwiesen, die Tenside aus anionischen, zwitterionischen oder nichtionischen Tensiden auszuwählen.

Als anionische Tenside eignen sich in erfindungsgemäßen Zubereitungen alle für die Verwendung am menschlichen Körper geeigneten anionischen oberflächenaktiven Stoffe. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 10 bis 22 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 oder 3 C-Atomen in der Alkanolgruppe,
- lineare Fettsäuren mit 10 bis 22 C-Atomen (Seifen),
- Ethercarbonsäuren der Formel R-O-(CH₂-CH₂O)ₓ -CH₂-COOH, in der R eine lineare Alkylgruppe mit 10 bis 22 C-Atomen und x = 0 oder 1 bis 16 ist,
- Acylsarcoside mit 10 bis 18 C-Atomen in der Acylgruppe,
- Acyltauride mit 10 bis 18 C-Atomen in der Acylgruppe,
- Acylisethionate mit 10 bis 18 C-Atomen in der Acylgruppe,
- Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen,
- lineare Alkansulfonate mit 12 bis 18 C-Atomen,
- lineare Alpha-Olefinsulfonate mit 12 bis 18 C-Atomen,
- Alpha-Sulfofettsäuremethylester von Fettsäuren mit 12 bis 18 C-Atomen,
- Alkylsulfate und Alkylpolyglykolethersulfate der Formel R-O(CH₂-CH₂O)ₓ-SO₃H, in der R eine bevorzugt lineare Alkylgruppe mit 10 bis 18 C-Atomen und x = 0 oder 1 bis 12 ist,
- Gemische oberflächenaktiver Hydroxysulfonate gemäß DE-A-37 25 030,
- sulfatierte Hydroxyalkylpolyethylen- und/oder Hydroxyalkylenpropylenglykolether gemäß DE-A-37 23 354,
- Sulfonate ungesättigter Fettsäuren mit 12 bis 24 C-Atomen und 1 bis 6 Doppelbindungen gemäß DE-A-39 26 344,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül sowie insbesondere Salze von gesättigten und insbesondere ungesättigten C8-C22-Carbonsäuren, wie Ölsäure, Stearinsäure, Isostearinsäure und Palmitinsäure.

Als zwitterionische Tenside werden solche oberflächenaktiven Verbindungen bezeichnet, die im Molekül mindestens eine quartäre Ammoniumgruppe und mindestens eine -COO⁽⁻⁾- oder -SO₃⁽⁻⁾-Gruppe tragen. Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethylammoniumglycinate, beispielsweise das Kokosacylaminopropyldimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der CTFA-Bezeichnung Cocamidopropyl Betaine bekannte Fettsäureamid-Derivat.

Unter ampholytischen Tensiden werden solche oberflächenaktiven Verbindungen verstanden, die außer einer C₈₋₁₈-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C₁₂₋₁₈-Acylsarcosin.

Nichtionische Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂₋₂₂-Fettsäuremono- und -diester von Anlagerungsprodukten von 1 bis 30 Mol Ethylenoxid an Glycerin,
- C₈₋₂₂-Alkylmono- und -oligoglycoside und deren ethoxylierte Analoga,
- Anlagerungsprodukte von 5 bis 60 Mol Ethylenoxid an Rizinusöl und gehärtetes Rizinusöl,
- Anlagerungeprodukte von Ethylenoxid an Sorbitanfettsäureester
- Anlagerungsprodukte von Ethylenoxid an Fettsäurealkanolamide.

Beispiele für die in den erfindungsgemäßen Haarbehandlungsmitteln verwendbaren kationischen Tenside sind insbesondere quartäre Ammoniumverbindungen. Bevorzugt sind Ammoniumhalogenide wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid. Weitere erfindungsgemäß verwendbare kationische Tenside stellen die quaternisierten Proteinhydrolysate dar.

Erfindungsgemäß ebenfalls geeignet sind kationische Silikonöle wie beispielsweise die im Handel erhältlichen Produkte Q2-7224 (Hersteller: Dow Corning; ein stabilisiertes Trimethylsilylamodimethicon), Dow Corning 929 Emulsion (enthaltend ein hydroxylamino-modifiziertes Silicon, das auch als Amodimethicone bezeichnet wird), SM-2059 (Hersteller: General Electric), SLM-55067 (Hersteller: Wacker) sowie Abil®-Quat 3270 und 3272 (Hersteller: Th. Goldschmidt; diquaternäre Polydimethylsiloxane, Quaternium-80).

Alkylamidoamine, insbesondere Fettsäureamidoamine wie das unter der Bezeichnung Tego Amid®S 18 erhältliche Stearylamidopropyldimethylamin, zeichnen sich neben einer guten konditionierenden Wirkung speziell durch ihre gute biologische Abbaubarkeit aus.

Ebenfalls sehr gut biologisch abbaubar sind quaternäre Esterverbindungen, sogenannte "Esterquats", wie die unter dem Warenzeichen Stepantex® vertriebenen Dialkylammoniummethosulfate und Methyl-hydroxyalkyldialkoyloxyalkyl-ammoniummethosulfate.

Ein Beispiel für ein als kationisches Tensid einsetzbares quaternäres Zuckerderivat stellt das Handelsprodukt Glucquat®100 dar, gemäß CTFA-Nomenklatur ein "Lauryl Methyl Gluceth-10 Hydroxypropyl Dimonium Chloride".

Bei den als Tenside eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so daß man Substanzgemische mit unterschiedlichen, vom jeweiligen Rohstoff abhängigen Alkylkettenlängen erhält.

Bei den Tensiden. die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite. Erdalkalimetallsalze von Ethercarbonsäuren, Erdalkalimetalloxide, hydroxide oder -alkoholate als Katalysatoren verwendet werden. Die Verwendung von Produkten mit eingeengter Homologenverteilung kann bevorzugt sein.

Weitere Wirk-, Hilfs- und Zusatzstoffe sind beispielsweise
- nichtionische Polymere wie beispielsweise Vinylpyrrolidon/Vinylacrylat-Copolymere, Polyvinylpyrrolidon und Vinylpyrrolidon/vinylacetat-copolymere und Polysiloxane,
- kationische Polymere wie quaternisierte Celluloseether, Polysiloxane mit quaternären Gruppen, Dimethyldiallylammoniumchlorid-Polymere, Acrylamid-Dimethyldiallylammoniumchlorid-Copolymere, mit - Diethylsulfat quaternierte Dimethylaminoethylmethacrylat-Vinylpyrrolidon-Copolymere, Vinylpyrrolidonlmidazoliniummethochlorid-Copolymere und quaternierter Polyvinylalkohol,
- zwitterionische und amphotere Polymere wie beispielsweise Acrylamidopropyltrimethylammoniumchlorid/Acrylat-Copolymere und Octylacrylamid/Methylmethacrylat/tert.Butylaminoethylmethacrylat/2-Hydroxypropylmethacrylat-Copolymere,
- anionische Polymere wie beispielsweise Polyacrylsäuren, vernetzte Polyacrylsäuren, Vinylacetat/Crotonsäure-Copolymere, Vinylpyrrolidon/Vinylacrylat-Copolymere, Vinylacetat/Butylmaleat/Isobornylacrylat-Copolymere, Methylvinylether/Maleinsäureanhydrid-Copolymere und Acrylsäure/Ethylacrylat/Ntert.Butylacrylamid-Terpolymere.
- Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate. z. B. Methylcellulose, Hydroxyalkylcellulose und Carboxymethylcellulose. Stärke-Fraktionen und Derivate wie Amylose, Amylopektin und Dextrine, Tone wie z. B. Bentonit oder vollsynthetische Hydrokolloide wie z.B. Polyvinylalkohol,
- Strukturanten wie Glucose und Maleinsäure,
- haarkonditionierende Verbindungen wie Phospholipide, beispielsweise Sojalecithin, Ei-Lecitin und Kephaline, sowie Silikonöle,
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quaternisierte Proteinhydrolysate,
- Parfümöle, Dimethylisosorbid und Cyclodextrine,
- Lösungsvermittler wie Ethanol, Isopropanol, Ethylenglykol, Propylenglykol, Glycerin und Diethylenglykol,
- Antischuppenwirkstoffe wie Piroctone Olamine und Zink Omadine,
- weitere Substanzen zur Einstellung des pH-Wertes,
- Wirkstoffe wie Panthenol, Pantothensäure, Allantoin, Pyrrolidoncarbonsäuren und deren Salze, Pflanzenextrakte und Vitamine,
- Cholesterin,
- Lichtschutzmittel,
- Konsistenzgeber wie Zuckerester, Polyolester oder Polyolalkylether,
- Fette und Wachse wie Walrat, Bienenwachs, Montanwachs, Paraffine, Fettalkohole und Fettsäureester.
- Fettsäurealkanolamide,
- Komplexbildner wie EDTA, NTA und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Trübungsmittel wie Latex,
- Perlglanzmittel wie Ethylenglykolmono- und -distearat,
- Treibmittel wie Propan-Butan-Gemische, N₂O, Dimethylether, CO₂ und Luft sowie
- Antioxidantien.

Die Bestandteile des wasserhaltigen Trägers werden zur Herstellung der erfindungsgemäßen Färbemittel in für diesen Zweck üblichen Mengen eingesetzt; z.B. werden Emulgiermittel in Konzentrationen von 0,5 bis 30 Gew.-% und Verdickungsmittel in Konzentrationen von 0.1 bis 25 Gew.-% des gesamten Färbemittels eingesetzt.

Die oxidative Entwicklung der Färbung kann grundsätzlich mit Luftsauerstoff erfolgen. Bevorzugt wird jedoch ein chemisches Oxidationsmittel eingesetzt, besonders dann, wenn neben der Färbung ein Aufhelleffekt an menschlichem Haar gewünscht ist. Als Oxidationsmittel kommen Persulfate, Chlorite und insbesondere Wasserstoffperoxid oder dessen Anlagerungsprodukte an Harnstoff, Melamin sowie Natriumborat in Frage. Weiterhin ist es möglich, die Oxidation mit Hilfe von Enzymen durchzuführen. Dabei können die Enzyme zur Übertragung von Luftsauerstoff auf die Entwicklerkomponente oder zur Verstärkung der Wirkung geringer Mengen vorhandener Oxidationsmittel dienen. Ein Beispiel für ein enzymatisches Verfahren stellt das Vorgehen dar, die Wirkung geringer Mengen (z.B. 1 % und weniger, bezogen auf das gesamte Mittel) Wasserstoffperoxid durch Peroxidasen zu verstärken.

Zweckmäßigerweise wird die Zubereitung des Oxidationsmittels unmittelbar vor dem Haarefärben mit der Zubereitung aus den Oxidationsfarbstoffvorprodukten vermischt. Das dabei entstehende gebrauchsfertige Haarfärbepräparat sollte bevorzugt einen pH-Wert im Bereich von 6 bis 10 aufweisen. Besonders bevorzugt ist die Anwendung der Haarfärbemittel in einem schwach alkalischen Milieu. Die Anwendungstemperaturen können in einem Bereich zwischen 15 und 40 °C liegen. Nach einer Einwirkungszeit von ca. 30 Minuten wird das Haarfärbemittel durch Ausspülen von dem zu färbenden Haar entfernt. Das Nachwaschen mit einem Shampoo entfällt, wenn ein stark tensidhaltiger Träger, z.B. ein Färbeshampoo, verwendet wurde.

Die nachfolgenden Beispiele sollen den Erfindungsgegenstand näher erläutern.

### Beispiele

### 1. Synthesebeispiele

### 1.1. Synthese von N,N'-Bis(4-aminophenyl)-1,4-diazacycloheptan 4 HCl

### 1. Stufe: N,N'-Bis(4-nitrophenyl)-1,4-diazacycloheptan

Eine Mischung, bestehend aus 22 g 1,4-Diazacycloheptan, 28,2 g p-Fluornitrobenzol und 300 ml Ethanol, wurde im Autoklaven 6 Stunden auf 150 °C erhitzt. Nach dem Abkühlen wurde der ausgefallene Feststoff abgesaugt. Das Produkt fiel in Form gelber Kristalle mit einem Schmelzpunkt von > 310 °C an.

### 2. Stufe: N,N'-Bis(4-aminophenyl)-1,4-diazacycloheptan 4HCl

14,8 g des Produktes aus Stufe 1 wurden in 300 ml Ethanol/Wasser-Mischung (1:1) suspendiert. Danach wurde mit einem Wasserstoffdruck von 20 atm. unter Verwendung eines üblichen Katalysators (Palladium auf Kohle) bei einer Temperatur von 50°C hydriert, bis keine Wasserstoffaufnahme mehr erfolgte. Nach dem Abkühlen wurde der Katalysator abfiltriert, die Lösung mit verdünnter Salzsäure angesäuert und bis zur Trockne eingedampft. Das Produkt fiel in Form hell-violetter Kristalle mit einem Schmelzpunkt von 196 °C (Zers.) an.

### 2.1. Synthese von N,N'-Bis(4-amino-2-methyl-phenyl)-1,4-diazacycloheptan 4HCl

### 1. Stufe: N,N'-Bis(4-nitro-2-methyl-phenyl)-1,4-diazacycloheptan

Eine Mischung, bestehend aus 7,9 g 1,4-Diazacycloheptan, 24,1 g 2-Fluor-5-nitro-toluol und 300 ml Ethanol, wurde im Autoklaven 6 Stunden auf 150 °C erhitzt. Nach dem Abkühlen wurde der ausgefallene Feststoff abgesaugt. Das Produkt fiel in Form gelber Kristalle mit einem Schmelzpunkt von 154 °C an.

### 2. Stufe: N,N'-Bis(4-amino-2-methyl-phenyl)-1,4-diazacycloheptan 4 HCl

3,5 g des Produktes aus Stufe 1 wurden in 400 ml Ethanol suspendiert. Danach wurde mit einem Wasserstoffdruck von 5 atm. unter Verwendung eines üblichen Katalysators (Palladium auf Kohle) bei einer Temperatur von 50°C hydriert, bis keine Wasserstoffaufnahme mehr erfolgte. Nach dem Abkühlen wurde der Katalysator abfiltriert, die Lösung mit verdünnter Salzsäure angesäuert und bis zur Trockne eingedampft. Das Produkt fiel in Form farbloser Kristalle mit einem Schmelzpunkt von ca. 220 °C (Zers.) an.

### 3.1. Synthese von N.N'-Bis(4-amino-3-methyl-phenyl)-1,4-diazacycloheptan 4 HCl

### 1. Stufe: N,N'-Bis(4-nitro-3-methyl-phenyl)-1,4-diazacycloheptan

Eine Mischung, bestehend aus 16,6 g 1,4-Diazacycloheptan, 52 g 5-Fluor-2-nitro-toluol und 300 ml Ethanol, wurde im Autoklaven 6 Stunden auf 150 °C erhitzt. Nach dem Abkühlen wurde der ausgefallene Feststoff abgesaugt. Das Produkt fiel in Form gelber Kristalle mit einem Schmelzpunkt von 214 °C an.

### 2. Stufe: N,N'-Bis(4-amino-3-methyl-phenyl)-1,4-diazacycloheptan 4 HCl

3,5 g des Produktes aus Stufe 1 wurden in 400 ml Ethanol suspendiert. Danach wurde mit einem Wasserstoffdruck von 5 atm. unter Verwendung eines üblichen Katalysators (Palladium auf Kohle) bei einer Temperatur von 50°C hydriert, bis keine Wasserstoffaufnahme mehr erfolgte. Nach dem Abkühlen wurde der Katalysator abfiltriert, die Lösung mit verdünnter Salzsäure angesäuert und bis zur Trockne eingedampft. Das Produkt fiel in Form beige-farbiger Kristalle mit einem Schmelzpunkt von 182 °C (Zers.) an.

### 2. Ausfärbungen

Es wurde zunächst eine Cremebasis folgender Zusammensetzung hergestellt [alle Angaben sind, soweit nicht anders vermerkt, in g]:

| | |
|---|---|
| Talgfettalkohol | 17,0 |
| Lorol®techn.¹ | 4,0 |
| Texapon®N 28² | 40,0 |
| Dehyton®K³ | 25,0 |
| Eumulgin®B 2⁴ | 1,5 |
| destilliertes Wasser | 12,5 |

| | |
|---|---|
| ¹ C₁₂₋₁₈-Fettalkohol (HENKEL) | |
| ² Natriumlaurylethersulfat (ca. 28 % Aktivsubstanz; CTFA-Bezeichnung: Sodium Laureth Sulfate) (HENKEL) | |
| ³ Fettsäureamid-Derivat mit Betainstruktur der Formel R-CONH(CH₂)₃N⁺(CH₃)₂CH₂COO⁻ (ca. 30 % Aktivsubstanz; CTFA-Bezeichnung Cocoamidopropyl Betaine) (HENKEL) | |
| ⁴ Cetylstearylalkohol mit ca. 20 Mol EO (CTFA-Bezeichnung: Ceteareth-20) (HENKEL) | |

Auf Basis dieser Creme wurde dann folgende Haarfärbecremeemulsion hergestellt:

| | |
|---|---|
| Cremebasis | 50,0 |
| Entwicklerkomponente | 7,5 mmol * |
| Kupplerkomponente | 7,5 mmol * |
| Na₂SO₃ (Inhibitor) | 1,0 |
| (NH₄)₂SO₄ | 1,0 |
| konz. Ammoniaklösung | ad pH 10 |
| Wasser | ad 100 |

| | |
|---|---|
| * sofern nicht anders vermerkt | |

Die Bestandteile wurden der Reihe nach miteinander vermischt. Nach Zugabe der Oxidationsfarbstoffvorprodukte und des Inhibitors wurde zunächst mit konzentrierter Ammoniaklösung der pH-Wert der Emulsion auf 10 eingestellt, dann wurde mit Wasser auf 100 g aufgefüllt.

Die oxidative Entwicklung der Färbung wurde mit 3 %iger Wasserstoffperoxidlösung als Oxidationslösung durchgeführt. Hierzu wurden 100 g der Emulsion mit 50 g Wasserstoffperoxidlösung (3 %ig) versetzt und vermischt.

Die Färbecreme wurde auf ca. 5 cm lange Strähnen standardisierten, zu 90 % ergrauten, aber nicht besonders vorbehandelten Menschenhaars aufgetragen und dort 30 Minuten bei 32 °C belassen. Nach Beendigung des Färbeprozesses wurde das Haar gespült, mit einem üblichen Haarwaschmittel gewaschen und anschließend getrocknet.
Für die Ausfärbungen wurden folgende Kuppler- und Entwickler-Komponenten verwendet:

Entwickler-Komponenten
- N,N'-Bis(4-aminophenyl)-1,4-diazacycloheptan (E1)
- N,N'-Bis(4-aminophenyl)-piperidin (E2) -Vergleichsbeispiel-
- N.N'-Bis(4-amino-2-methyl-phenyl)-1,4-diazacycloheptan (E3)
- N,N'-Bis(4-amino-3-methyl-phenyl)-1,4-diazacycloheptan (E4)
- 2,4,5,6-Tetraaminopyrimidin (E5)
- 4-Amino-3-methylphenol (E6)
- 2-Aminomethyl-4-aminophenol (E7)
- p-Aminophenol (E8)
- 1-(2-Hydroxyethyl)-2,5-diaminobenzol (E9)
- 4-Hydroxy-2,5,6-triaminopyrimidin (E10)
- N,N-Bis(2-hydroxyethyl)-p-phenylendiamin (E11)
- p-Toluylendiamin (E12)
- 2-(2,5-Diaminophenoxy)-ethanol (E13).

Kuppler-Komponenten
- 1-Naphthol (K1)
- Resorcin (K2)
- 2-Methyl-5-aminophenol (K3)
- 1,3-Bis(2,4-diaminophenoxy)propan (K4)
- 2-Chlor-6-methyl-3-aminophenol (K5)
- 3-Amino-6-methoxy-2-methylaminophenol (K6)
- 2-Hydroxy-4-aminophenoxyethanol (K7)
- 2-Amino-3-hydroxy-pyridin (K8)
- 4-Chlorresorcin (K9)
- 2,4-Diaminophenoxyethanol (K10)
- 2,6-Dimethyl-3-amino-phenol (K11)
- 2,4-Dichlor-3-amino-phenol (K12)
- 3,4-Methylendioxy-phenol (K13)
- 2-Methyl-resorcin (K14)
- m-Aminophenol (K15)
- 2-Methyl-5-(2-hydroxyethylamino)-phenol (K16)
- 2.6-Dihydroxy-3.4-dimethylpyridin (K17)
- 3-Amino-2-methylamino-6-methoxy-pyridin (K18)
- 1-Methoxy-2-amino-4-(2-hydroxyethylamino)-benzol (K19)
- 2.6-Bis(2-hydroxyethylamino)-toluol (K20)
- 1,7-Dihydroxynaphthalin (K21)
- 2,7-Dihydroxynaphthalin (K22).

Es wurden folgende Ausfärbungen gefunden:

| Entwickler | Kuppler | Nuance des gefärbten Haares | Intensität |
|---|---|---|---|
| E1 | K1 | lasurblau | sehr intensiv |
| E2 | K1 | graumagenta | sehr schwach |
| E1 | K2 | dunkelbraun | intensiv |
| E2 | K2 | dunkelblond | schwach |
| E1 | K3 | dunkelviolett | intensiv |
| E2 | K3 | rotbraun | sehr schwach |
| E1 | K4 | schwarzblau | sehr intensiv |
| E2 | K4 | dunkelviolett | sehr schwach |
| E1 | K5 | dunkelviolett | sehr intensiv |
| E2 | K5 | violett | sehr schwach |
| E1 | K7 | dunkelviolett | intensiv |
| E1 | K8 | dunkelviolett | intensiv |
| E1 | K9 | graubraun | intensiv |
| E1 | K10 | schwarzblau | intensiv |
| E1 | K11 | tiefviolett | intensiv |
| E1 | K12 | schwarzblau | intensiv |
| E1 | K13 | graubraun | intensiv |
| E1 | K14 | dunkelbraun | intensiv |
| E1 | K15 | schwarzblau | intensiv |
| E1 | K16 | dunkelviolett | intensiv |
| E1 | K17 | graublau | intensiv |
| E1 | K18 | dunkelgrün | intensiv |
| E1 | K19 | schwarzblau | intensiv |
| E3 | K1 | mattrot | intensiv |
| E3 | K5 | graurot | intensiv |
| E4 | K4 | dunkelblau | intensiv |
| E4 | K5 | schwarzblau | intensiv |
| E4 | K6 | dunkeltürkis | intensiv |
| E1+E5^{a} | K4+K14^{a} | dunkelblau | intensiv |
| E1+E6^{a} | K8 | rotbraun | intensiv |
| E1+E7^{a} | K3 | photobraun | intensiv |
| E1+E8^{a} | K3 | photobraun | intensiv |
| E1+E9^{a} | K3 | dunkelviolett | intensiv |
| E1+E10^{a} | K20 | schwarzblau | intensiv |
| E1+E11^{a} | K2+K15^{a} | blaugrau | intensiv |
| E1+E8^{a} | K4+K9^{a} | marineblau | intensiv |
| E1+E10^{a} | K11+K21^{a} | dunkelviolett | intensiv |
| E1+E12^{a} | K18+K22^{a} | nordischblau | intensiv |
| E1+E6^{a} +E13^{a} | K3^{b} | dunkelrubin | intensiv |

| | | | |
|---|---|---|---|
| ^{a} jeweils 0,375 mmol | | | |
| ^{b} 0,113 mmol | | | |

## Patentansprüche

1. 1,4-Diazacycloheptan-Derivate der allgemeinen Formel (I), 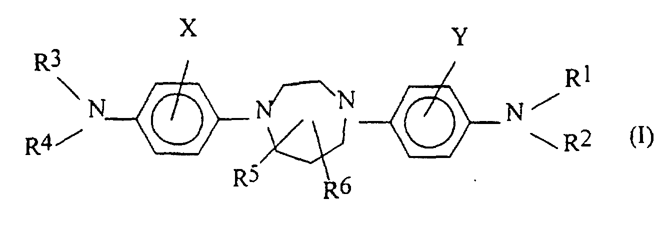 in der
- R¹, R², R³ und R⁴ unabhängig voneinander stehen für Wasserstoff, eine C₁₋₄-Alkyl- oder Hydroxyalkylgruppe oder eine C₂₋₄-Dihydroxyalkylgruppe,
- X und Y unabhängig voneinander stehen für Wasserstoff, Chlor, Fluor, eine C₁₋₄-Alkyl-, -Hydroxyalkyl-, -Aminoalkyl- oder -Alkoxygruppe, eine C₂₋₄-Dihydroxyalkylgruppe oder eine Allylgruppe und
- R⁵ und R⁶ unabhängig voneinander stehen für Wasserstoff oder eine C₁₋₄-Alkylgruppe.

2. 1,4-Diazacycloheptan-Derivate nach Anspruch 1, **dadurch gekennzeichnet, daß** beide Reste R⁵ und R⁶ für Wasserstoff stehen.

3. 1,4-Diazacycloheptan-Derivate nach Anspruch 1 oder 2, **dadurch gekennzeichnet, daß** mindestens drei der Gruppen R¹, R², R³ und R⁴ für Wasserstoff stehen.

4. 1,4-Diazacycloheptan-Derivate nach einem der Ansprüche 1 bis 3, **dadurch gekennzeichnet, daß** X und Y unabhängig voneinander stehen für Wasserstoff, Fluor, Chlor oder eine C₁₋₄-Alkyl-, insbesondere Methylgruppe.

5. 1,4-Diazacycloheptan-Derivate nach Anspruch 1, **dadurch gekennzeichnet, daß** sie aus N,N'-Bis(4-aminophenyl)-1,4-diazacycloheptan und N,N'-Bis(4-amino-3-methyl-phenyl)-1,4-diazacycloheptan ausgewählt sind.

6. Verwendung von 1,4-Diazacycloheptan-Derivaten nach einem der Ansprüche 1 bis 5 und deren wasserlöslichen Salzen als Entwickler-Komponente in Oxidationshaarfärbemitteln.

7. Oxidationsfärbemittel zum Färben von Keratinfasern, enthaltend Kuppler-Komponenten und Entwickler-Komponenten in einem wasserhaltigen Träger, **dadurch gekennzeichnet, daß** es als Entwickler-Komponente ein 1,4-Diazacycloheptan-Derivat nach einem der Ansprüche 1 bis 5 enthält.

8. Oxidationsfärbemittel nach Anspruch 7, **dadurch gekennzeichnet, daß** es als Kuppler-Komponente 1-Naphthol, Resorcin, 1,3-Bis(2,4-diaminophenoxy)propan, 2-Hydroxy-4-aminophenoxyethanol, 4-Chlorresorcin, 2,4-Diaminophenoxyethanol, 2-Methyl-resorcin, 2-Methyl-5-(2-hydroxyethylamino)-phenol, 3-Amino-2-methylamino-6-methoxy-pyridin enthält.

9. Oxidationsfärbemittel nach Anspruch 7 oder 8, **dadurch gekennzeichnet, daß** es neben den 1,4-Diazacycloheptan-Derivaten mindestens eine weitere Entwickler-Komponente enthält.

10. Oxidationsfärbemittel nach Anspruch 9, **dadurch gekennzeichnet, daß** die weitere Entwickler-Komponente ausgewählt ist aus 2,4,5,6-Tetraaminopyrimidin, 4-Hydroxy-2,5,6-triaminopyrimidin, 1-(2-Hydroxyethyl)-2,5-diaminobenzol, p-Phenylendiamin, p-Toluylendiamin, p-Aminophenol, 3-Methyl-p-aminophenol, 2-Aminomethyl-p-aminophenol und 1,3-N,N'-Bis(2'-hydroxyethyl)-N,N'-bis(4'-amino-phenyl)-diamino-1,3-propan-2-ol.

11. Oxidationsfärbemittel nach einem der Ansprüche 7 bis 10, **dadurch gekennzeichnet, daß** Entwicklerkomponenten in einer Menge von 0,005 bis 20 Gew.-%, vorzugsweise 0,1 bis 5 Gew.-%, und Kupplerkomponenten in einer Menge von 0,005 bis 20 Gew.-%, vorzugsweise 0,1 bis 5 Gew.-%, jeweils bezogen auf das gesamte Oxidationsfärbemittel, enthalten sind.

12. Oxidationsfärbemittel nach einem der Ansprüche 7 bis 10, **dadurch gekennzeichnet, daß** weiterhin mindestens ein direktziehender Farbstoff enthalten ist.

## Claims

1. 1,4-Diazacycloheptane derivatives corresponding to general formula (I): 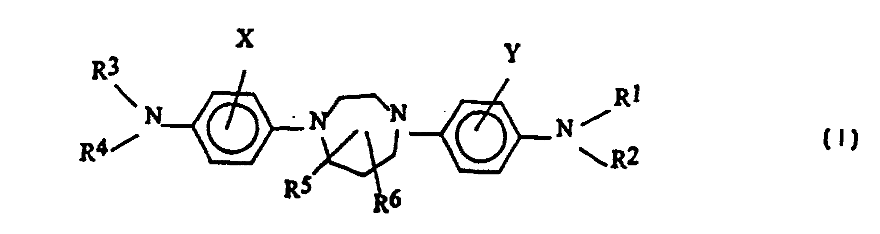 in which
- R¹, R², R³ and R⁴ independently of one another represent hydrogen, a C₁₋₄ alkyl or hydroxyalkyl group or a C₂₋₄ dihydroxyalkyl group,
- X and Y independently of one another represent hydrogen, chlorine, fluorine, a C₁₋₄ alkyl, hydroxyalkyl, aminoalkyl or alkoxy group, a C₂₋₄ dihydroxyalkyl group or an allyl group and
- R⁵ and R⁶ independently of one another represent hydrogen or a C₁₋₄ alkyl group.

2. 1,4-Diazacycloheptane derivatives as claimed in claim 1, **characterized in that** both substituents R⁵ and R⁶ are hydrogens.

3. 1,4-Diazacycloheptane derivatives as claimed in claim 1 or 2, **characterized in that** at least three of the substituents R¹, R², R³ and R⁴ are hydrogens.

4. 1,4-Diazacycloheptane derivatives as claimed in any of claims 1 to 3, **characterized in that** X and Y independently of one another represent hydrogen, fluorine, chlorine or a C₁₋₄ alkyl group more particularly a methyl group.

5. 1,4-Diazacycloheptane derivatives as claimed in claim 1, **characterized in that** they are selected from N,N'-bis-(4-aminophenyl)-1,4-diazacycloheptane and N,N'-bis-(4-amino-3-methylphenyl)-1,4-diazacycloheptane.

6. The use of the 1,4-diazacycloheptane derivatives claimed in any of claims 1 to 5 and water-soluble salts thereof as a primary intermediate in oxidation hair colorants.

7. An oxidation colorant for coloring keratin fibers containing secondary intermediates and primary intermediates in a water-containing carrier, **characterized in that** it contains a 1,4-diazacycloheptane derivative according to any of claims 1 to 5 as primary intermediate.

8. An oxidation colorant as claimed in claim 7, **characterized in that** it contains 1-naphthol, resorcinol, 1,3-bis-(2,4-diaminophenoxy)-propane, 2-hydroxy-4-aminophenoxyethanol, 4-chlororesorcinol, 2,4-diaminophenoxyethanol, 2-methyl resorcinol, 2-methyl-5-(2-hydroxyethylamino)-phenol, 3-amino-2-methylamino-6-methoxypyridine as secondary intermediate.

9. An oxidation colorant as claimed in claim 7 or 8, **characterized in that**, in addition to the 1,4-diazacycloheptane derivatives, it contains at least one other primary intermediate.

10. An oxidation colorant as claimed in claim 9, **characterized in that** the other primary intermediate is selected from 2,4,5,6-tetraaminopyrimidine, 4-hydroxy-2,5,6-triaminopyrimidine, 1-(2-hydroxyethyl)-2,5-diaminobenzene, p-phenylenediamine, p-toluylenediamine, p-aminophenol, 3-methyl-p-aminophenol, 2-aminomethyl-p-aminophenol and 1,3-N,N'-bis-(2'-hydroxyethyl)-N,N'-bis-(4'-aminophenyl)-diamino-1,3-propan-2-ol.

11. An oxidation colorant as claimed in any of claims 7 to 10, **characterized in that** primary intermediates are present in a quantity of 0.005 to 20% by weight and preferably 0.1 to 5% by weight while secondary intermediates are present in a quantity of 0.005 to 20% by weight and preferably 0.1 to 5% by weight, based on the oxidation colorant as a whole.

12. An oxidation colorant as claimed in any of claims 7 to 10, **characterized in that** at least one substantive dye is additionally present.

## Revendications

1. Dérivés de 1,4-diazacycloheptane de formule générale I : 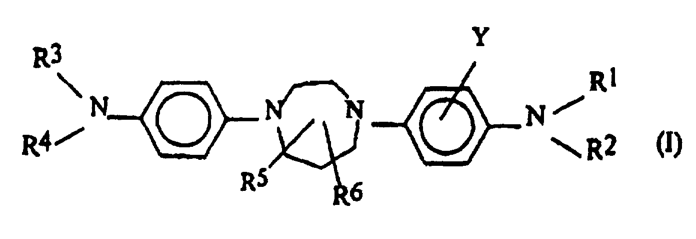 dans laquelle,
R¹, R², R³ et R⁴ indépendamment l'un de l'autre, représentent de l'hydrogène, un groupe alkyle ou hydroxyalkyle en C₁-C₄ ou un groupe dihydroxyalkyle en C₂-C₄,
X et Y indépendamment l'un de l'autre représentent de l'hydrogène, du chlore, du fluor, un groupe alkyle- hydroxyalkyle-, aminoalkyle- ou alkoxy- en C₁-C₄, un groupe dihydroxyalkyle en C₂-C₄ ou un groupe allyle
et,
R⁵ et R⁶ indépendamment l'un de l'autre représentent de l'hydrogène ou un groupe alkyle en C₁-C₄.

2. Dérivés de 1,4-diazacycloheptane selon la revendication 1,
**caractérisé en ce que**
les deux restes R⁵ et R⁶ représentent de l'hydrogène.

3. Dérivés de 1,4-diazacycloheptane selon l'une quelconque des revendications 1 ou 2,
**caractérisé en ce qu'**
au moins trois des groupes R¹, R², R³ et R⁴ représentent de l'hydrogène.

4. Dérivés de 1,4-diazacycloheptane selon l'une quelconque des revendications 1 à 3
**caractérisé en ce que**
X et Y indépendamment l'un de l'autre, représentent de l'hydrogène, du fluor, du chlore ou un alkyle en C₁-C₄, en particulier le groupe méthyle.

5. Dérivés de 1,4-diazacycloheptane selon la revendication 1,
**caractérisé en ce qu'**
ils sont choisis dans le groupe du N,N'-bis(4-aminophényl)-1,4-diazacycloheptane et N,N'-bis(4-amino-3-méthylphényl)-1,4-diazacycloheptane.

6. Utilisation de dérivés du 1,4-diazacycloheptane selon l'une quelconque des revendications 1 à 5, et leurs sels solubles dans l'eau comme composants d'agent de développement dans des teintures pour cheveux par oxydation.

7. Colorant par oxydation pour la teinture des fibres de kératine, contenant des composants d'agent de couplage et des composants d'agent de développement dans un support contenant de l'eau,
**caractérisé en ce qu'**
il contient comme composant d'agent de développement un dérivé de 1,4-diazacycloheptane selon l'une quelconque des revendications 1 à 5.

8. Colorant par oxydation selon la revendication 7,
**caractérisé en ce qu'**
il contient comme composant d'agent de couplage du 1-naphtol, du résorcinol, du 1,3-bis(2,4-diaminophenoxy)propane, le 2-hydroxy-4-aminophénoxyéthanol, le 4-chlororésorcinol, le 2,4-diaminophénoxyéthanol, le 2-méthylrésorcinol, le 2-me-thyl-5-(2-hydroxyéthylamino)phénol, la 3-amino-2-méthylamino-6-méthoxypyridine.

9. Colorant par oxydation selon l'une quelconque des revendications 7 ou 8,
**caractérisé en ce qu'**
il contient à côté des dérivés de 1,4-diazacycloheptane au moins un autre composant d'agent de développement.

10. Colorant par oxydation selon la revendication 9,
**caractérisé en ce qu'**
l'autre composant d'agent de développement est choisi parmi la 2,4,5,6-tétraaminopyrimidine, la 4-hydroxy-2,5,6-triaminopyrimidine, le 1-(2-hydroxyéthyl)2,5-diaminobenzène, la p-phénylènediamine, la p-toluylènediamine, le p-aminophénol, le 3-métyl-p-aminophénol, le 2-aminométhyl-p-aminophénol et le 1,3-N,N'-bis(2'-hydroxyéthyl)N,N'-bis(4'-aminophényl)diamino-1,3-propane-2-ol.

11. Colorant par oxydation selon l'une quelconque des revendications 7 à 10,
**caractérisé en ce que**
des composants d'agent de développement sont présents en une quantité de 0,005 à 20 % en poids, de préférence de 0,1 à 5 % en poids, et des composants d'agent de couplage en une quantité de 0,005 à 20 % en poids de préférence de 0,1 à 5 % en poids, à chaque fois rapporté à la totalité du colorant par oxydation.

12. Colorant par oxydation selon l'une quelconque des revendications 7 à 10,
**caractérisé en ce qu'**
en outre au moins un colorant à action directe est présent.
